# EUROPEAN PATENT SPECIFICATION

(11) **EP 1 377 329 B1**
(45) Date of publication and mention of the grant of the patent: **21.10.2009**
(21) Application number: 02747269.5
(22) Date of filing: 09.04.2002
(51) Int. Cl.: A61M 5/32

(54) **SAFETY INJECTION DEVICE FOR A LIQUID OR SEMI-SOLID COMPOSITION**
SICHERHEITSINJEKTIONSVORRICHTUNG FÜR EINE FLÜSSIGE ODER HALBFESTE ZUSAMMENSETZUNG
DISPOSITIF D'INJECTION DE SECURITE POUR COMPOSITION LIQUIDE OU SEMI-SOLIDE

(30) Priority: 10.04.2001 US 282765 P
(43) Date of publication of application: 07.01.2004
(73) Proprietor: IPSEN PHARMA, 92100 Boulogne-Billancourt (FR)
(72) Inventor: CHERIF-CHEIKH, Roland, E-08860 Castelldefels (ES)
(74) Representative: Bourgouin, André
(86) International application number: PCT/EP2002/004008
(87) International publication number: WO 2002/096488

(56) References cited:
- EP-A- 1 090 652
- WO-A-00/56383
- WO-A-98/29152
- US-A- 5 658 259
- US-A- 6 110 147

## Description

### BACKGROUND OF THE INVENTION

The invention relates to a device for the parenteral administration through a needle of liquid or semi-solid drug compositions wherein the needle is protected before and after the injection.

The parenteral introduction of pharmaceutically active compounds is preferred over oral dosage for many indications, e.g. where the drug to be administered would partially or totally degrade in the gastrointestinal tract or where there is need for rapid biological response. The need for extemporaneous preparation of such parenteral compositions is eliminated, or simplified, by the use of pre-filled administration devices in which the liquid to be injected is pre-loaded into the device (e.g. a pre-loaded syringe). Such pre-loaded devices, however, have a number of drawbacks, including the inability to preserve the asepsis or sterility of the needle, as well as the general danger of using an exposed needle. To eliminate these drawbacks, it is necessary to avoid the direct exposure of the needle with the environment both prior to and following injection. Examples of injection devices can be found in prior art (see for examples devices described in Patent applications N°WO 98/29152 or N° WO 00/56383).

### SUMMARY OF THE INVENTION

The invention features a comparatively inexpensive injection device with a needle for parenteral injection of liquid or semi-solid drug compositions into a subject, e.g. a mammal such as a human, wherein the needle is protected before and after the injection.

In general, the invention features an injection device including a housing, the housing having proximal and distal ends and designed to contain a liquid or semi-solid drug composition; a hollow needle, the needle affixed to the distal end of the housing and extending longitudinally within the housing; a plunger, the plunger arranged to slide within the proximal end of the housing; and a hollow sleeve, the hollow sleeve arranged to cover the needle prior to injection and to retract into the housing during injection; wherein the device is designed such that when the sleeve is pressed against the subject, the sleeve retracts into the housing and the drug composition is delivered through the needle and into the subject.

In one embodiment, the device is further designed such that when the drug composition is forced from the housing, the plunger forces the sleeve out of the housing to cover the needle. In a further embodiment, the housing contains the liquid or semi-solid drugs composition.

In another embodiment, the device further comprises a septum plunger, the septum plunger slidably arranged within the housing between the plunger and the distal end of the housing. In a further embodiment, the device is configured such that when the drug composition is forced from the housing, the plunger forces the septum plunger into the sleeve, and the septum plunger forces the sleeve out of the housing to cover the needle. In still a further embodiment, the housing contains the liquid or semi-solid drug composition between the plunger and the septum plunger.

In still another embodiment, the housing contains a liquid and a dry drug composition, where the device is designed to combine the liquid and the dry drug composition prior to injection.

In a further embodiment the device comprises a releasable lock which inhibits the movement of the plunger into the housing. In a still further embodiment the device comprises a removable cap which covers the sleeve. In yet a still further embodiment the proximal end of the housing comprises a flange and/or the plunger comprises a flange.

The device comprises a cartridge or tube, said cartridge or tube comprising a distal end, said distal end closed by a cap, seal or septum; a proximal end, said proximal end closed by a plunger tip slidably arranged within the cartridge or tube; and a reservoir between said cap, seal or septum and said plunger tip.

Optionally said cap, seal or septum may be attached to said distal end with a classical clip means, e.g., using a metal ring. The cap, seal or septum and the plunger tip each is made of a suitable material, i.e., a material compatible with the intended use of the injection device. In a preferred embodiment the cap, seal or septum and the plunger tip each independently is made of a non-rigid solid material such as rubber, polybromobutyl, or the like. In a more preferred embodiment the cap, seal or septum and the plunger tip each is made of the same material.

The cartridge or tube is configured to contain a liquid or semi-solid drug composition within the reservoir and is introduced into the housing of the device, e.g., through the proximal end of the housing. The cartridge or tube is further configured such that it can be moved within the housing, e.g., toward or away from the proximal end of the needle. Said cartridge or tube is optionally of a standard variety.

The injection device is configured such that, after the cartridge or tube is connected to the proximal end of the needle, i.e., after the proximal end of the needle pierces the cap, seal or septum located at the distal end of the cartridge or tube, then when the sleeve is pressed against the subject the sleeve retracts into the housing thereby exposing the distal end of the needle and allowing the distal end of the needle to penetrate the subject. Thereafter, when the plunger tip at the proximal end of the cartridge or tube is urged into the cartridge or tube, i.e., toward the distal end of the cartridge or tube, the drug composition is urged from the cartridge or tube through the needle and into the subject.

According to a particular variant of this invention, the cartridge or tube further comprises a proximal compartment located toward the proximal end of the cartridge or tube and a distal compartment located toward the distal end of the cartridge or tube, wherein the proximal compartment and the distal compartment are separated by a plunger. In this variant said proximal compartment contains a liquid component of a composition and the distal compartment contains a solid component of said composition. In this variant the device is configured such that, in operation, the liquid and solid components are mixed prior to injection.

In one embodiment, the device is further configured such that when the drug composition is forced from the cartridge or tube, the cartridge or tube urges the sleeve out of the housing thereby covering the needle after the injection and, optionally, urging the withdrawal of the needle from the subject. In a further embodiment, the cartridge or tube contains the liquid or semi-solid drug composition.

In another embodiment, the cartridge or tube further comprises a septum cap or seal to close the distal end and a septum plunger to close the proximal end of the tube. Said septum cap or seal is fixed by a clip and said septum plunger is slidably arranged within the cartridge or tube. In a further embodiment, the device is configured such that, when the cartridge or tube is urged sufficiently into the housing the proximal end of the needle passes through the septum cap or seal, and, when the drug composition is urged from the cartridge or tube by the septum plunger, said septum plunger urges the cartridge or tube and the cartridge or tube urges the sleeve out of the housing to cover the needle. In still a further embodiment, the cartridge or tube contains the liquid or semi-solid drug composition between the septum cap or seal and the septum plunger.

In still another embodiment, the cartridge or tube contains a liquid and a dry drug composition, where the device is designed to combine the liquid and the dry drug composition prior to injection.

The device can further include a cartridge or tube locking means to inhibit the movement of the cartridge or tube in the housing, e.g., after the cartridge or tube has been connected to the needle. The proximal end of the housing may have a flange and the plunger may also have a flange.

In still another embodiment of the injection device the housing comprises the reservoir and the hollow needle is affixed to the distal end of the reservoir and extends only longitudinally outside said reservoir. A housing or protection sleeve is configured on the plunger and is arranged to slide around the reservoir. The device is configured such that when the drug composition is urged from the reservoir, the plunger housing covers said reservoir. At the end of the injection the plunger is released from the plunger housing, e.g., by the proximal end of the reservoir, and the plunger slides into said plunger housing thereby allowing the plunger housing to cover the needle.

A further object of the invention is therefore an injection device for injecting liquid or semisolid composition into a subject, the device comprising: a reservoir having a proximal and distal end, said distal end being configured to contain a liquid or semi-solid composition; a hollow needle, said needle affixed to the distal end of the reservoir and extending longitudinally outside said reservoir; a plunger arranged to slide within the proximal end of the reservoir; said plunger arranged to retract after injection into a plunger housing slidably connected to the proximal end of the reservoir and arranged to cover the plunger, the reservoir and the needle after injection; wherein the device is designed such that when the plunger housing is pushed around the reservoir, the plunger is pushed into the reservoir, the composition is pushed from the reservoir through the needle and into the subject.

According to a preferred execution mode of this injection device, said plunger housing is disconnected from said plunger due to a release mechanism into said proximal end of the reservoir. According to another preferred execution mode, the protection sleeve is designed to be locked in an irreversible manner by, e.g., mechanical means once the needle has been protected.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the preferred methods and materials are described below. All publications, patents, patent applications, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### BRIEF DESCRIPTION OF THE DRAWINGS

FIG. **1** is a partial cross-sectional view of an injection device prior to use.
FIG. **2** is a partial cross-sectional view of the injection device of FIG. 1 during use.
FIG. **3** is a partial cross-sectional view of the device with the needle injected into a subject.
FIG. **4** is a partial cross-sectional view of the injection device being withdrawn from the subject with the drug composition remaining in the subject.
FIG. **5** is a partial cross-sectional view of the injection device following complete withdrawal of the needle from the subject.
FIG. **6** is a partial cross-sectional view of the injection device through line 6-6 in FIG. 1.
FIG. **7** is a view of the sleeve of the injection device.
FIG. **8** is a partial cross-sectional view of a cartridge injection device prior to pre-introduction of the cartridge.
FIG. **9** is a partial cross-sectional view of a cartridge injection device prior to use.
FIG. **10** is a cross-sectional view of the cartridge injection device of FIG. **9** during use after connection of the cartridge to the needle.
FIG. **11** is a partial cross-sectional view of the cartridge injection device of FIG. **10** during use after plunger setting.
FIG. **12** is a cross-sectional view of the cartridge injection device with the needle injected into a subject.
FIG. **13** is a partial cross-sectional view of the cartridge injection device being withdrawn from the subject with a drug composition remaining in the subject.
FIG. **14** is a partial cross-sectional view of the cartridge injection device following complete withdrawal of the needle from the subject.
FIG. **15** is a cross-section of the injection device through line **6-6** in FIG. **8****.**
FIG. **16** is a cross-section of a cartridge containing a liquid or semi-solid composition.
FIG. **17** is a cross-section of a cartridge containing a liquid and a dry drug:
   - 17A: prior to rehydration;
   - 17B: after rehydration.
FIG. **18** is a cross-section of the housing with the two options or the cartridge (right) and the tube (left).
FIG. **19** is a cross-section of the tube containing a liquid and a dry drug before and after rehydration.
FIG. **20** is a cross-section view at the stage of FIG. **10** in a case where tube containing a liquid and a dry drug before and after rehydration.
FIG **21** is a cross-section view of a reservoir injection device prior to use.
FIG **22** is a cross-sectional view of the reservoir injection device after drug composition injection.
FIG **23** is a cross-sectional view of the reservoir injection device following complete withdrawal of the needle from the subject.

### DETAILED DESCRIPTION

It is believed that one skilled in the art can, based on the description used herein, utilize the present invention to its fullest extent. The following specific embodiments are, therefore, to be construed as merely illustrative, and not limiting.

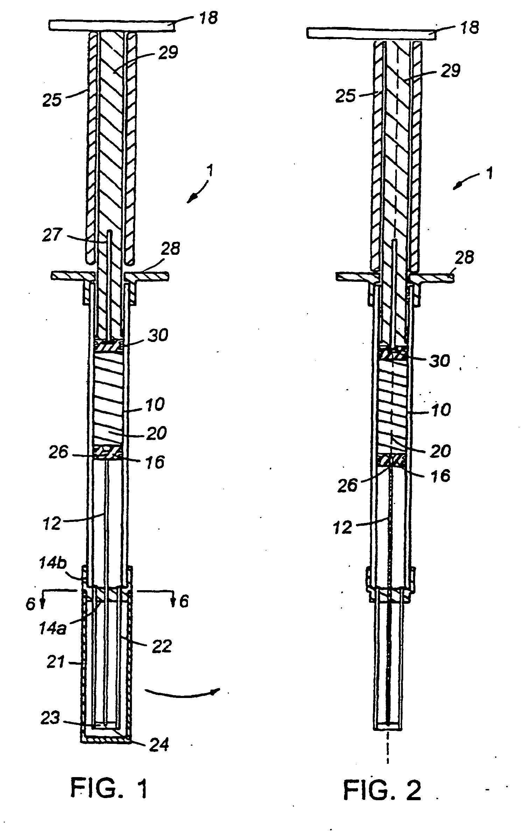

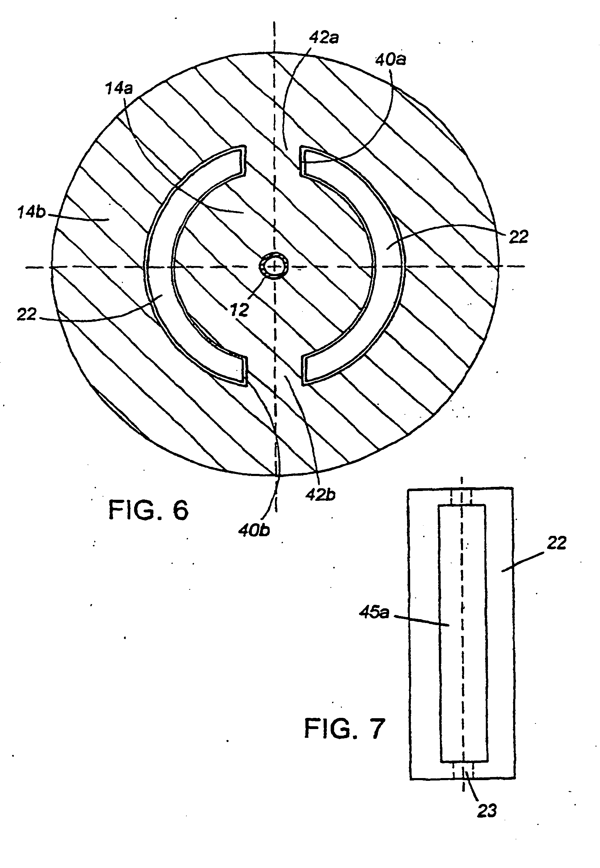

FIG. 1 shows injection device **1** including housing **10,** having a proximal end and a distal end **14***a*, **14***b*. The distal end of the housing **10** has two holes **40***a* and **40***b* partially separating the two parts **14***a* and **14***b* of said distal end (as best seen in FIG. **6****).** Needle **12** is attached to part **14***a* of the distal end. The housing **10** can be made from a suitably rigid material such as glass, plastic, metal, and the like. The needle **12** is hollow and double-ended, wherein its distal end, remaining outside housing **10,** has a point capable of piercing the skin of a subject, and its proximal end, remaining within housing **10,** is capable of piercing septum plunger **16.** On the proximal end of housing **10** is a flange **28** to assist in removal of device **1** from the subject following injection.

A sleeve **22** surrounds needle **12** so that needle **12** is not fully exposed to the environment until used. Sleeve **22** has longitudinal slots **45***a* and **45***b* along its length (see FIG. 7; slot **45***b* is on the back of the sleeve and is thus not shown). The two parts **14***a* and **14***b* of the distal end are joined by radially extending connecting members **42***a* and **42***b* (see FIG. **6****).** Connecting members **42***a* and **42***b*, respectively, slide through slots **45***a* and **45***b* in sleeve **22,** while sleeve **22** slides through holes **40***a* and **40***b* in housing **10.** Sleeve **22** can be made of suitably rigid material, such as metal, glass, plastic, and the like. Seal **24** covers the opening **23** of sleeve **22** to maintain the sterility of needle **12** and prevent sleeve **22** from unintentionally retracting into housing **10** through holes **40***a* and **40***b* prior to injection.

Seal **24** can be made of a thin material, such as plastic or wax, which is easily penetrated by needle **12** during injection. A similar seal can also cover slots **45***a* and **45***b* in sleeve **22,** to further protect the sterility of needle **12.**

Septum plunger **16,** contained within housing 10, includes a bore **26,** in which needle **12** rests prior to subsequently piercing septum plunger **16.** A liquid or semi-solid composition **20** is isolated in housing **10** between the septum plunger **16** and the plunger tip **30,** attached to plunger **29.** Septum plunger **16** and plunger tip **30** may be made of non-rigid, solid material such as rubber, polybromobutyl, and the like, which allows septum plunger **16** and plunger tip **30** to slide within housing **10** but still maintain sufficient friction with the inner sides of housing **10** to seal composition **20** within housing **10.**

The proximal end of plunger **29** has a thumb flange **18** to assist in the depression of plunger **29** into housing **10,** and the distal end of plunger **29** has a longitudinal bore **27** to receive needle **12** following injection of composition **20** out and through needle **12.** Plunger **29** can be made from a suitably rigid material, such as glass, metal, plastic, and the like. A removable lock **25** may be placed between flange **18** and flange **28** to inhibit further depression of plunger **29** into housing **10** after activation of the device **1,** i.e. after the housing **10** is filled with a drug composition and the proximal end of the needle is pierced through septum plunger **16.** A removable cap **21** can also be used to protect both needle **12** and sleeve **22** prior to use. Both cap **21** and lock **25** can be made from suitably rigid material such as plastic, metal, rubber, and the like.

FIG. **2** shows device **1** wherein plunger **29** has been pressed into housing **10** to activate device **1** as follows. When plunger **29** is depressed, plunger tip **30,** composition **20,** and septum plunger **16** are displaced towards the distal end of housing **10.** Septum plunger **16** is pierced at bore **26** by needle **12.** As a result, the proximal end of needle **12** is exposed to composition **20.** Device **1** is now in an activated state. Lock **25,** by contacting both flange **18** and flange **28,** inhibits the further displacement of composition **20** from housing **10** to needle **12** following activation of device **1,** i.e, composition **20** is allowed to fill needle **12,** but lock **25** inhibits significant release of composition **20** through needle **12.**

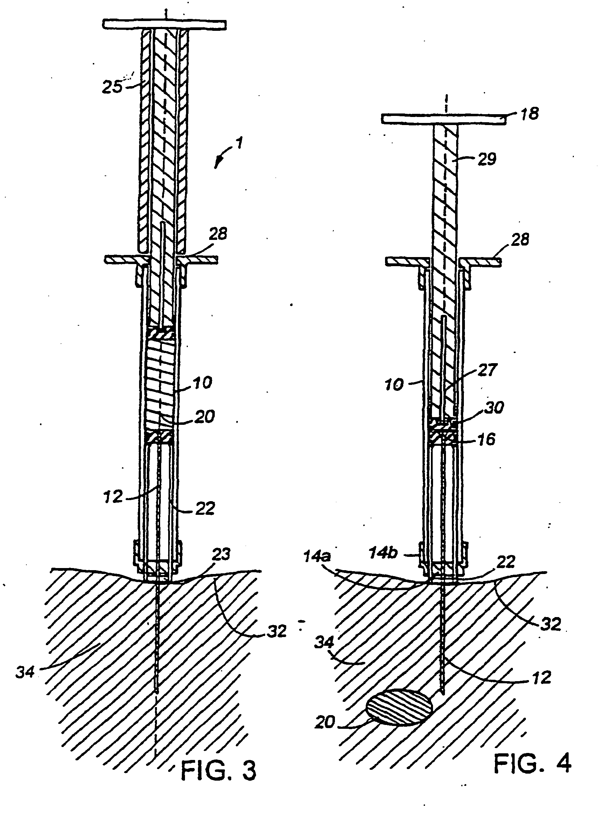

FIG. **3** shows device **1** wherein needle **12** has penetrated skin **32** of the subject being treated. As device **1** is pressed against skin **32,** sleeve **22** is retracted into housing **10,** through holes **40***a* and **40***b***,** by the force of pressure against skin **32.** Needle **12** passes through sleeve **22** at opening **23.** As shown, needle **12** has penetrated through skin **32** into the subcutaneous layer **34.**

FIG. **4** shows device **1** wherein lock **25** has been removed and plunger **29** has been depressed, which moves plunger tip **30** toward septum plunger **16,** thereby injecting composition **20** into subcutaneous layer **34** through needle **12.** Once composition **20** has been injected and plunger tip **30** rests against septum plunger **16,** housing **10** is moved away from skin **32** by exerting pressure against the lower part of the flange **28** while simultaneously exerting oppositing pressure on flange **18** of plunger **29.** This relative movement of the plunger **29** and housing **10** causes plunger tip **30** to force septum plunger **16** against sleeve **22** as both plunger tip **30** and septum plunger **16** slide towards parts **14***a* and **14***b* of the distal end of housing **10,** which in turn forces sleeve **22** out of housing **10** through holes **40***a* and **40***b*. As plunger tip **30** and septum plunger **16** are moved toward distal end of housing **10,** needle **12** penetrates septum plunger **16,** plunger tip **30,** and enters bore **27** in plunger **29.**

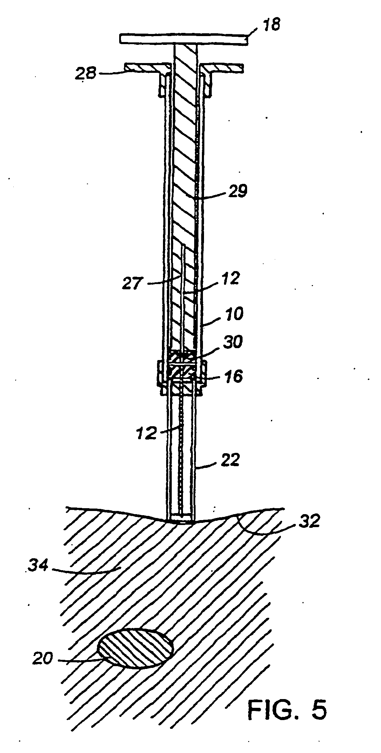

FIG. **5** shows needle **12** fully withdrawn from skin **32** and sleeve **22** fully covering needle **12.** Composition **20** remains in the subcutaneous layer of the patient. As can also be seen in FIG. **5****,** the proximal end of needle **12** has been pushed through septum plunger **16** and plunger tip **30** and remains in bore **27** of plunger **29.**

FIG. **6** is a cross-sectional view of FIG. 1 at **6****-6.** FIG. **6** shows holes **40***a* and **40***b* in housing **10.** Radially extending connecting members **42***a* and **42***b* extend through slots **45***a* and **45***b***,** respectively, to connect parts **14***a* and **14***b* of the distal end. Needle **12** is fixed to central part **14***a* of the distal end, and sleeve **22** can slide through holes **40***a* and **40***b****.***

FIG. 7 shows an isolated sleeve **22** having slots **45***a* and **45***b* **(*****45**b* is not shown but positioned directly opposite to slot **45***a* on the other side of sleeve **22)** and opening **23.** Radially extending connecting members **42***a* and **42***b*, respectively, slide through slots **45***a* and **45***b***.**

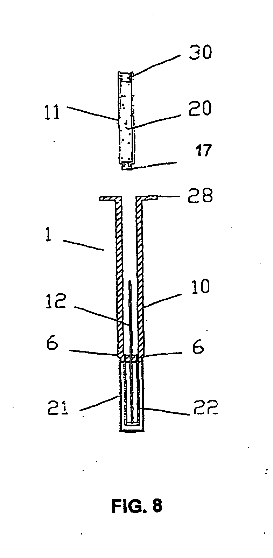

FIG. **8** shows a cartridge injection device 1 including a housing **10** having a proximal and a distal end.

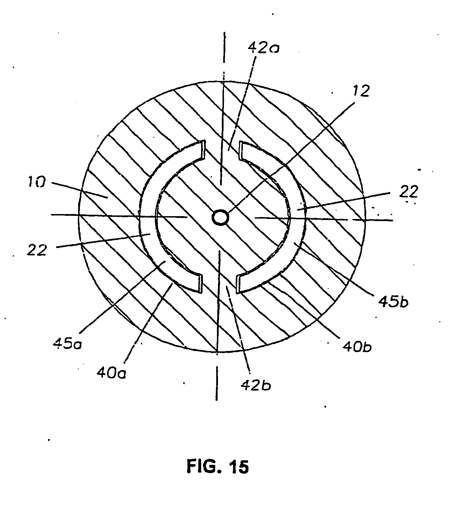

The distal end of housing **10** has at least one hole and for example two holes, **40***a* and **40b,** partially separating the two parts of the distal end (as best seen in FIG. **15****).** Needle **12** is attached to the distal end. The housing **10** can be made from a suitably rigid material such as glass, plastic, metal, and the like. The needle **12** is hollow and double-ended, wherein its distal end, remaining outside housing **10,** has a point capable of piercing the skin of a subject, and its proximal end remaining within housing **10** is capable of piercing septum cap **17** and septum plunger **30** of the cartridge or tube **11.** On the proximal end of housing **10** is a flange **28** to assist in injection. The removal of device 1 from the subject following injection is assisted by extension of sleeve **22.**

A sleeve **22** surrounds needle **12** so that needle **12** is not fully exposed to the environment until used. Sleeve **22** has at least one longitudinal slot along its length, and for example two, **45**a and **45**b (see FIG**. 15****).** The two parts of the distal end are joined by radially extending connecting members **42**a and **42**b (see **FIG. 15****).** Connecting members **42**a and **42**b respectively slide through slots **45**a and **45**b in sleeve **22,** while sleeve **22** slides through holes **40**a and **40**b in housing **10.** Sleeve **22** can be made of suitably rigid material such as glass, plastic, metal and the like.

A seal **24** (not shown) on needle **12** can be made of a thin material, such as a plastic sheet, a plastic packaging material or a bag which is easily penetrated by needle **12** during injection. A similar seal can also cover the sleeve **22** to further protect the sterility of the needle **12.**

Septum plunger **16**, contained within housing **10,** can include a bore **26** in which needle **12** rests prior to subsequently piercing septum plunger **16.** A liquid or semi-solid composition **20** is isolated in the cartridge **11** between septum plunger **16** and plunger tip **30.** Septum plunger **16** and plunger tip **30** may be made of non-rigid solid material such as rubber, polybromobutyl, or the like, which allows plunger tip **30** to slide within cartridge **11** but maintaining sufficient friction with the inner sides of the cartridge **11** to seal composition **20** within cartridge **11.**

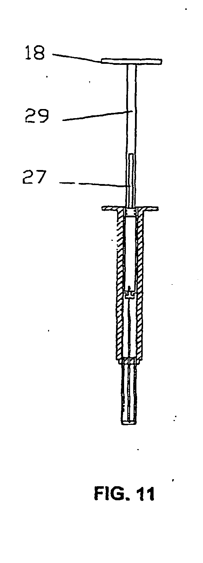

The proximal end of plunger **29** has a thumb flange **18** to assist in the depression of plunger **29** into cartridge **11** and the distal end of plunger **29** has a longitudinal bore **27** to receive needle **12** following injection of composition **20** out and through needle **12** (see FIG. **11****).** Plunger **29** can be made from a suitably rigid material such as glass, plastic, metal and the like. A removable lock **25** may be placed between flange **18** and flange **28** to inhibit the further depression of plunger **29** into housing **10** after activation of device **1,** i.e. after the housing **10** is filled with a tube or a cartridge and the proximal end of the needle is pierced through septum cap **17.**

A removable cap **21** can also be used to protect both needle **12** and sleeve **22** prior to use. Both cap **21** and lock **25** can be made from suitably rigid material such as plastic, metal, rubber, and the like

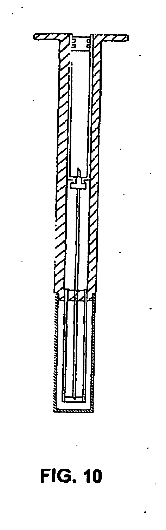

Septum cap **17** on distal end of cartridge **11** is sealed, e.g., with a metal ring (not shown). A liquid or semi-solid composition **20** is isolated in cartridge or tube **11** between the septum cap **17** and the plunger tip **30,** which will be attached to plunger **29** (see FIG. **11****).** The cartridge or tube **11** can be connected to the proximal end of needle **12** by applying pressure to the proximal end of the cartridge or tube, e.g., by pushing with the thumb (see FIG. **10****).**

Septum cap **17** and plunger tip **30** may be made of non-rigid material such as rubber, polybromobutyl, and the like, which allows needle **12** to pierce septum cap **17** and plunger tip **30,** and allows plunger tip **30** to slide sealably within cartridge or tube **11.**

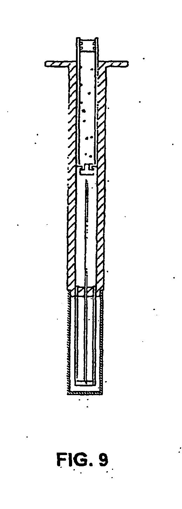

FIG. **9** shows, during use of device **1,** the introduction of cartridge or tube **11** into the housing **10.**

FIG. **10** shows the connection of cartridge or tube **11** on needle **12** through septum cap **17.**

FIG. **11** shows settlement of plunger **29** on plunger tip **30.**

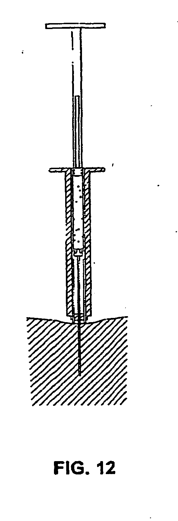

FIG. **12** shows injection of needle **12** with retraction of sleeve **22** into housing **10.**

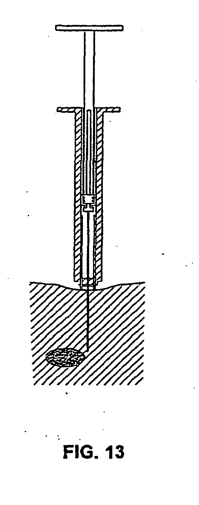

FIG. **13** shows injection of composition **20** into the tissue, e.g., using thumb on plunger flange **18** and the other fingers on housing flange **28.**

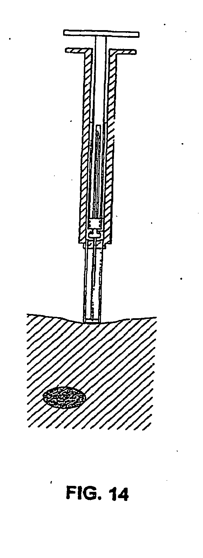

FIG. **14** shows removal of the device from the subject following injection, where cartridge or tube **11** urges sleeve **22** from housing **10** around needle **12** and, consequently, removes needle **12** from body tissue.

FIG. **15** shows a cross-section, through line **6-6** shown in FIG. **8****,** of distal end **14**b of housing **10** with two apertures or holes **40**a and **40**b through which slots **45**a and **45**b of sleeve **22** slide. Two connecting members **42**a and **42**b separate the holes and connect external part of the housing **10** with the internal part where needle **12** is fixed.

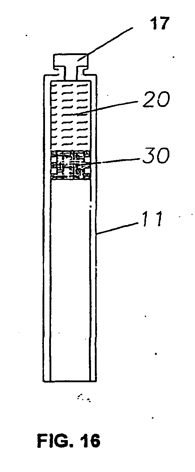

FIG. **16** shows a cartridge or tube **11** used in the housing **10** of device **1** with composition **20** between septum cap **17** and plunger tip **30.**

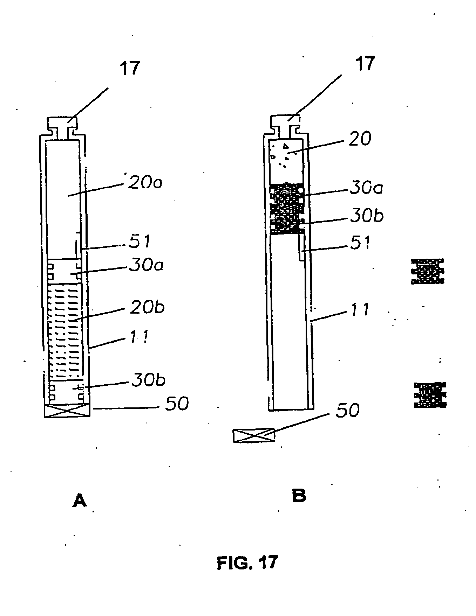

FIG. **17A** shows a cartridge or tube **11** used in the housing **10** of device **1** with a releasable lock **50.** The liquid part of the composition **20B** is loaded in cartridge or tube **11** between two septum plunger **30A** and septum plunger **30B.** Septum plunger **30A** is placed into cartridge or tube **11** just before by-pass **51.** Septum plunger **30B** is locked with the lock **50** in contact with cartridge or tube **11.** The solid part of the composition **20A** is loaded, e.g., under vacuum, in cartridge or tube **11** between septum plunger **30A** and septum cap **17.**

FIG.**17B** shows cartridge or tube **11** of FIG. **17A** after removal of the releasable lock **50.** The composition **20** is prepared by passage of the liquid part of the composition **20B** through the by-pass **51** into the solid part under vacuum.

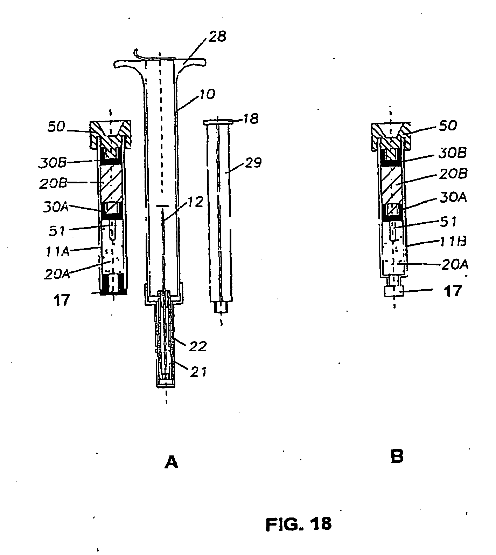

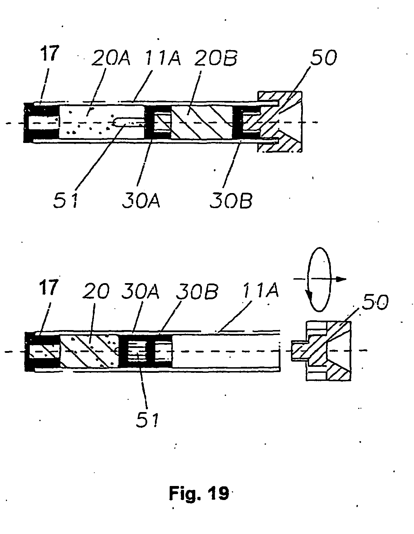

FIG. **18** shows device **1** with the tube option **11A** or the cartridge option **11B** presented with the dual chamber arrangement of FIG. **17****.** The housing **10** can be the same for both options **(11A** or **11B).** Before removal of releasable lock **50,** tube **11A** or cartridge **11B** cannot be connected on needle **12** through septum cap **17.** After removal of releasable lock **50,** the rehydration is realized as described in FIG. **17** or FIG. **19** and tube **11A** or cartridge **11B** can be operably connected to needle **12.** The plunger **29** is attached to septum plunger **30B** and the infection is performed.

FIG. **19** shows, for tube **11A,** the rehydration process described in FIG.**17**, performed by removing lock **50.** Before removing lock **50** on FIG.**12A,** tube **11A** cannot be introduced into the housing deep enough to introduce needle **12** through septum cap **17** into the tube **11A** Releasable lock **50** also maintains plunger **30A** at the top of by-pass **51** despite the vacuum in the solid part of the composition **20A.** After removing lock **50** on FIG. **12B,** tube **11A** can be introduced into the housing to introduce needle **12** through septum cap **17** into the tube **11A.** Before this introduction by removing lock **50,** the composition **20** is prepared by mixing the solid part **20A** and the liquid part **20B** due to the rehydration obtained by the vacuum of the chamber containing solid part **20A.**

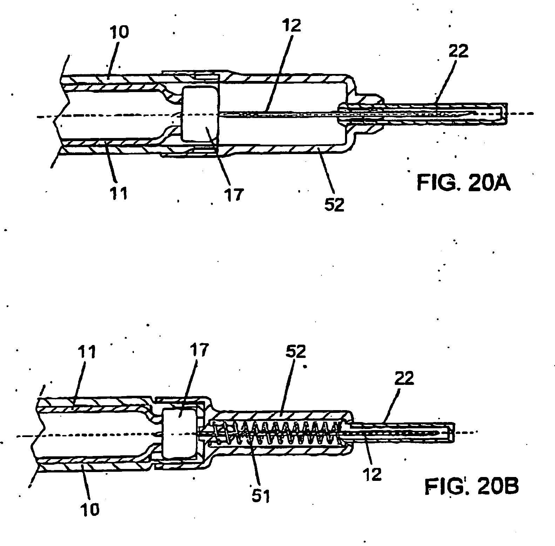

FIG. **20** shows **2** possible variations in the arrangement of FIG. **8** during use after connection of the cartridge or tube **11 on** the needle **12** when the needle **12** is not directly attached to the distal end of housing **10** but to a support **52** which corresponds to distal end of housing **10** in the way sleeve **22** is affixed to it.

This independent support **52** can be connected to housing **10,** e.g., like a pen or cartridge disposable needle is connected e.g., by screwing onto housing **10** after needle **12** is introduced through septum cap **17.**

In FIG. **20A****,** the removal of the device from the subject following injection is obtained by sleeve **22** as in FIG. **8** due to the displacement of cartridge or tube **11.**

FIG. **20B** shows another alternative where the extension of sleeve **22** after injection is facilitated by spring **51** without displacement of cartridge or tube **11.** This allows the thread to be a standard one and the device to be adapted on any other existing cartridge pen or syringe injector.

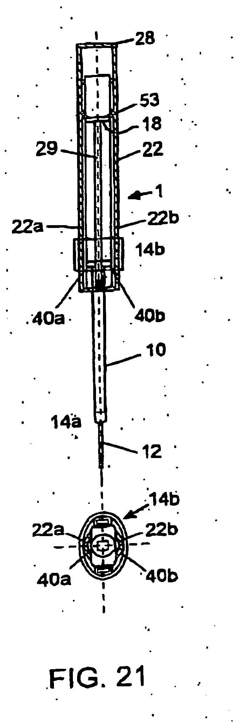

FIG. **21** shows injection device **1,** including reservoir **10,** having a distal end **14a** and a proximal end **14b.** The proximal end of the reservoir **10** has two holes **40a** and **40b** adapted to operatively accept arms **22a** and **22b.** Needle **12** is operatively attached to distal end **14a.** Reservoir **10** can be made from a suitably rigid material such as glass, plastic, metal, or the like. The needle **12** is hollow and single-ended outside the reservoir **10** with a tip capable of piercing the skin of a subject On the proximal end of housing is a flange **28** having, e.g., an elliptic shape, which assists both plunger movement in the reservoir and removal of device **1** from the subject via extension of sleeve **22** following injection.

In this embodiment the sleeve **22** comprises a plunger housing made of a suitably rigid material such as metal, glass, plastic, or the like. The plunger housing **22** surrounds plunger **29** so that when plunger **29** slides into reservoir **10,** plunger housing **22** slides around reservoir **10.** Plunger housing **22** has longitudinal slots (not shown) and arms **22a** and **22b** along its length. Arms **22a** and **22b** pass through holes **40a** and **40b** respectively, in the proximal end **14b.**

The proximal end of plunger **29** is covered by plunger housing **22** up to a flange **18.** Flange **18** assists depression of plunger **29** into reservoir **10** along with simultaneous depression of plunger housing **22** around reservoir **10** due to the removable lock **53** or connection means with plunger **29** into plunger horsing **22.**

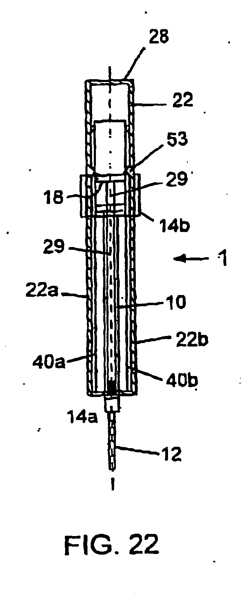

FIG. **22** shows device 1 as depicted in FIG. 21 wherein plunger **29** has been depressed by plunger housing **22** thereby injecting composition from reservoir **10** through needle **12.**

Once composition has been injected plunger **29** rests against the bottom or distal end of reservoir **10,** which is also completely covered by plunger housing **22.**

Reservoir **10** is then moved away from the injection needle site by exerting pressure against the lower part of the flange on proximal end **14b** of the reservoir **10** while simultaneously exerting opposing pressure on flange **28** of plunger housing **22.** This relative movement of the plunger housing **22** and reservoir **10** causes plunger **29** to be released from removable lock **53** due to sliding guide and release mechanism on reservoir proximal end **14b,** and plunger housing slides around reservoir **10** and needle **12,** which in turn urges needle **12** out of injection site.

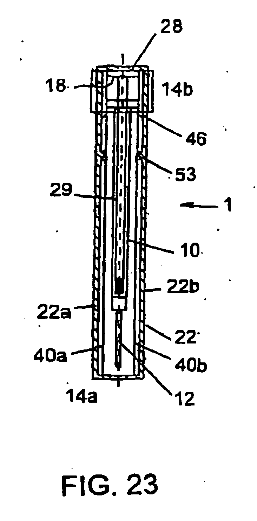

FIG. **23** shows needle **12** fully withdrawn from the injection site and plunger housing **22** covering reservoir **10** and needle **12.**

Flange **18** is equipped with means **46** to secure, optionally irreversibly, the plunger housing 22 once the needle **12** has been protected.

Composition **20** is a liquid or a semi-solid composition containing a drug. The drug of composition **20** can be any drug capable of being parenterally administered as a liquid or a semi-solid. For example, the drug can be a vaccine, a peptide, a protein, or a small chemical entity. Examples of suitable drugs include, e.g., insulin and heparin. For drugs which are not stable in liquids over an extended period of time, the liquid and the dry drug can be stored in separate chambers within housing **10.** The device can be configured such that the liquid and the dry drug are combined together just prior to injection.

For example, the chamber created between septum plunger **16** and plunger tip **30** (e.g. in FIG. **1****)** in housing **10** can be separated into two separate parts by a fixed wall or film that can be punctured, e.g. by pressure of the plunger **29** on the plunger tip **30,** or a puncturing means. Alternatively, the two parts of the chamber can be separated by a moving wall or septum. In this case, the top or proximal part of the chamber above the moving wall or septum contains the liquid portion of the composition, and the distal part of the chamber contains the solid, e.g., powder, portion of the composition. When plunger **29** is urged into housing **10,** it applies pressure to plunger tip **30** and plunger tip **30** applies pressure to the liquid portion of the composition. This, in turn, applies pressure on the moving septum, causing it to move in a distal direction. The housing is configured with a liquid bypass (e.g., a bulge or passage in the housing wall) in a location that initially prevents passage of liquid from one part of the chamber to the other, but when the moving septum reaches a specific location, the bypass allows the liquid to pass from the top or proximal part of the chamber into the lower or distal part of the chamber on the other side of the moving septum.

To maintain sterility, the device of the invention can be stored in a conventional blister pack or pouch prior to use.

### OTHER EMBODIMENTS

It is to be understood that while the invention has been described in conjunction with the detailed description thereof, the foregoing description is intended to illustrate and not limit the scope of the appended claims. Other aspects, advantages, and modifications are within the claims.

## Claims

1. An injection device (1) for injecting liquid or semi solid composition (20) into a subject, the device comprising:
a hollow housing (10) having a proximal end and a distal end (14a, 14b);
a hollow needle (12) having a proximal end and a distal end, said needle (12) affixed to the distal end of the housing (10) and said proximal end of said needle (12) extending longitudinally within said housing (10),
a sleeve (22) sliday connected to the distal end of the housing (10) arranged to cover the needle prior to injection and to retract into the housing (10) during injection;
**characterised in that:**
said injection device (1) comprises a cartrige or tube (11), said cartridge or tube (11) comprising a distal end and a proximal end, said distal end closed by a septum cap (17) and said proximal end closed by a plunger tip (30) slidably arranged within the cartridge or tube (21); and a reservoir between said septum cap (17) and said plunger tip (30); and said injection device (1) is configured such that when said cartridge or tube (11) is inserted into said housing (10) said septum cap (17) forms an operable connection with said proximal end of said hollow needle (12); whereby when the plunger tip (30) is urged into the cartridge or tube, the composition is urged from the cartridge or tube (11); through the needle (12) into the subject;
and said cartridge or tube (11) further comprising a proximal compartment, a distal compartment, and a bypass (51); said proximal compartment and said distal compartment separated by a first septum plunger slidably arranged with the cartridge or tube (11).

2. A device of claim 1, wherein the device (1) is further configured such that when the composition (20) is urged from the cartridge (11), the cartridge (11) urges the sleeve (22) to extend from the housing (10) to cover the needle (12) during and after the injection.

3. A device of claim 1, wherein the cartridge (11) contains the liquid or semi-solid composition (20) between the septum cap (17) and the septum plunger (30B).

4. A device of claim 1, wherein the device (1) further comprises a releasable lock (50) to inhibit the movement of the cartridge (11) within the housing (10).

5. A device (1) of claim 5, wherein said proximal compartment contains a liquid component (20b) a composition and said distal compartment contains a solid component (20a) of said composition (20), and wherein the device (1) is configured such that the liquid and solid components (20a, 20b) are mixed prior to injection.

6. A device (1) of claim 5 or 6, wherein said liquid component (20b) passes from said proximal compartment to said distal compartment through said bypass (51).

7. device (1) of claim 5, 6 or 7, wherein said distal chamber is maintained under vacuum.

8. A device (1) according to any of claims 1 to 8, wherein the distal end of the housing (10) is an independent distal end connected to said housing (10) prior to injection.

9. A device (1) of claim 9 wherein the independent distal end further comprises a spring (51), wherein said spring (51) urges the sleeve (22) of said distal end to cover the needle (12).

## Patentansprüche

1. Einspritzvorrichtung (1) für die Einspritzung einer flüssigen oder halbflüssigen Zusammensetzung (20) in ein Subjekt, bestehend aus:
einer hohlen Spritzenzylinder (10) mit einem proximalen Ende und einem distalen Ende (14a, 14b);
einer hohlen Nadel (12) mit einem proximalen und einem distalen Ende, wobei die genannte Nadel (12) am distalen Ende der Spritzenzylinder (10) angebracht wird und sich das genannte proximale Ende der genannten Nadel (12) in Längsrichtung in der genannten Spritzenzylinder (10) erstreckt.
einer Muffe (22), die gleitend am distalen Ende der Spritzenzylinder (10) angeschlossen und so angeordnet ist, dass sie die Nadel vor der Einspritzung öffnet und sich während der Einspritzung in die Spritzenzylinder (10) zurückzieht;
**dadurch gekennzeichnet, dass** die genannte Einspritzvorrichtung (1) folgendes umfasst:
eine Patrone oder Röhre (11), wobei die genannte Patrone oder Röhre (11) ein distales Ende und ein proximales Ende umfasst, wobei das distale Ende von einer Septumkappe (17) verschlossen ist und das genannte proximale Ende von einer Plungerspitze (30) verschlossen ist, die gleitend in der Patrone oder der Röhre (11) angeordnet ist; und ein Reservoir zwischen der genannten Septumkappe (17) und der genannten Plungerspitze (30);
und die genannte Einspritzvorrichtung (1) ist derart ausgebildet, dass wenn die genannte Patrone oder Röhre (11) in der genannten Spritzenzylinder (10) ist, die genannte Septumkappe (17) eine mit dem genannten proximalen Ende der genannten hohlen Nadel (12) bedienbare Verbindung bildet, wodurch die Zusammensetzung, wenn die Plungerspitze (30) in die Patrone oder die Röhre eingeschoben wird, von der Patrone oder der Röhre (11) aus durch die Nadel (12) und in das Subjekt geschoben wird; und die genannte Patrone oder Röhre (11) umfasst ferner eine proximale Kammer, eine distale Kammer und eine Derivation (51); wobei die proximale Kammer und die distale Kammer von einem ersten Septumtaucher getrennt sind, der gleitend in der Patrone oder der Röhre (11) angeordnet ist.

2. Vorrichtung gemäß Anspruch 1, bei der die Vorrichtung (1) ferner so ausgebildet ist, dass wenn die Zusammensetzung (20) aus der Patrone (11) geschoben wird, die Patrone (11) die Muffe (22) verschiebt, um sich von der Spritzenzylinder (10) aus zu erstrecken, um die Nadel (12) während und nach der Einspritzung abzudecken.

3. Vorrichtung gemäß Anspruch 1, bei der die Patrone (11) die flüssige oder halbflüssige Zusammensetzung (20) zwischen der Septumkappe (17) und dem Septumtaucher (30B) enthält.

4. Vorrichtung gemäß Anspruch 1, bei der die Vorrichtung (1) ferner eine Verriegelung (50) umfasst, die gelöst werden kann, um die Bewegung der Patrone (11) in der Spritzenzylinder (10) zu hemmen.

5. Vorrichtung (1) gemäß Anspruch 5, bei der die genannte proximale Kammer ein flüssiges Bestandteil (20b) einer Zusammensetzung enthält und die genannte distale Kammer ein festes Bestandteil (20a) der genannten Zusammensetzung (20) enthält, und bei der die Vorrichtung (1) so ausgebildet ist, dass das flüssige Bestandteil und das feste Bestandteil (20a, 20b) vor der Einspritzung gemischt werden.

6. Vorrichtung (1) gemäß Anspruch 5 oder 6, bei der das genannte flüssige Bestandteil (20b) über die genannte Derivation (51) von der proximalen Kammer in die genannte distale Kammer gelangt.

7. Vorrichtung (1) gemäß Anspruch 5, 6 oder 7, bei der die genannte distale Kammer unter Vakuum gehalten wird.

8. Vorrichtung (1) gemäß einem der Ansprüche 1 bis 8, bei der das distale Ende der Spritzenzylinder (10) ein unabhängiges distales Ende ist, das vor der Einspritzung an der genannten Spritzenzylinder (10) angeschlossen wird.

9. Vorrichtung (1) gemäß Anspruch 9, bei der das unabhängige distale Ende ferner eine Feder umfasst und bei der die Feder (51) die Muffe (22) des genannten distalen Endes verschiebt, um die Nadel (12) abzudecken.

## Revendications

1. Dispositif d'injection (1) pour injecter une composition liquide ou semi-solide (20) dans un sujet, le dispositif comprenant :
un corps de seringue (10) ayant une extrémité proximale et une extrémité distale (14a, 14b) ;
une aiguille creuse (12) ayant une extrémité proximale et une extrémité distale, ladite aiguille (12) étant apposée à l'extrémité distale du corps de seringue (10) et ladite extrémité proximale de ladite aiguille (12) s'étendant longitudinalement dans ledit corps de seringue (10).
un manchon (22) connecté de façon coulissante à l'extrémité distale du corps de seringue (10) et disposé pour couvrir l'aiguille avant l'injection et se rétracter dans le corps de seringue (10) pendant l'injection ;
**caractérisé en ce que** le dit dispositif d'injection (1) comprend :
une cartouche ou tube (11), ladite cartouche ou ledit tube (11) comprenant une extrémité distale et une extrémité proximale, la dite extrémité distale étant fermée par un capuchon à septum (17) et la dite extrémité proximale étant fermée par une extrémité du piston (30) disposée de façon coulissante dans la cartouche ou le tube (11) ; et un réservoir entre le dit capuchon à septum (17) et ladite extrémité du piston (30) ;
et ledit dispositif d'injection (1) est configuré de façon que lorsque ladite cartouche ou ledit tube (11) dans ledit corps de seringue (10), le dit capuchon à septum (17) forme une connexion opérable avec ladite extrémité proximale de ladite aiguille creuse (12), ce par quoi lorsque l'extrémité du piston (30) est poussée dans la cartouche ou le tube, la composition est poussée depuis la cartouche ou le tube (11), au travers de l'aiguille (12) et dans le sujet ; et ladite cartouche ou ledit tube (11) comprend en outre un compartiment proximal, un compartiment distal et une dérivation (51) ; ledit compartiment proximal et ledit compartiment distal étant séparés par un premier plongeur de septum disposé de façon coulissante dans la cartouche ou le tube (11).

2. Dispositif selon la revendication 1 dans lequel le dispositif (1) est en outre configuré de façon que lorsque la composition (20) est poussée depuis la cartouche (11), la cartouche (11) pousse le manchon (22) pour s'étendre depuis le corps de seringue (10) pour recouvrir l'aiguille (12) pendant et après l'injection.

3. Dispositif selon la revendication 1, dans lequel la cartouche (11) contient la composition liquide ou semi-solide (20) entre le piston (17) et le septum du piston (30B).

4. Dispositif selon la revendication 1 dans lequel le dispositif (1) comprend en outre un verrouillage libérable (50) pour empêcher le mouvement de la cartouche (11) dans le corps de seringue (10).

5. Dispositif (1) selon la revendication 5, dans lequel ledit compartiment proximal contient une partie liquide (20b) d'une composition et ledit compartiment distal contient la partie solide (20a) de ladite composition (20) et dans lequel le dispositif (1) est configuré de façon que le liquide et le solide (20a, 20b) soient mélangés avant injection.

6. Dispositif (1) selon la revendication 5 ou 6, dans lequel ledit liquide (20b) passe dudit compartiment proximal audit compartiment distal au travers de ladite dérivation (51).

7. Dispositif (1) selon la revendication 5, 6 ou 7 dans lequel ladite chambre distale est maintenue sous vide.

8. Dispositif (1) selon l'une quelconque des revendications 1 à 8, dans laquelle l'extrémité distale du corps de seringue (10) est une extrémité distale indépendante connectée audit corps de seringue (10) avant injection.

9. Dispositif (1) selon la revendication 9 dans laquelle l'extrémité distale indépendante comprend en outre un ressort (51) dans lequel le ressort (51) pousse le manchon (22) de ladite extrémité distale pour couvrir l'aiguille (12).
